Europäisches Patentamt

European Patent Office

Office européen des brevets

(11) Publication number: **0 123 294**
**B1**

# EUROPEAN PATENT SPECIFICATION

(45) Date of publication of patent specification: **03.01.90**

(51) Int. Cl.⁵: **C 12 N 15/00,** C 12 P 21/00

(21) Application number: **84104456.3**

(22) Date of filing: **19.04.84**

(54) **Yeast cell transformation vectors.**

(30) Priority: **22.04.83 US 487753**

(43) Date of publication of application:
**31.10.84 Bulletin 84/44**

(45) Publication of the grant of the patent:
**03.01.90 Bulletin 90/01**

(84) Designated Contracting States:
**AT BE CH DE FR GB IT LI LU NL SE**

(56) References cited:
**EP-A-0 035 781**
**EP-A-0 116 201**
**CELL, vol. 30, no. 3, October 1982, Cambridge, Mass. J. KURJAN et al. " Structure of a Yeast Pheromone Gene (MFalpha): A Putative alpha-Factor Precurser Contains Four Tandem Copies of Mature alpha-Factor" pages 933-943**
**CELL, vol. 32, no. 3, March 1983, Cambridge, Mass.D.JULIUS et al. " Yeast alpha Factor is Processed from a Larger Precursor Polypeptide: The Essential Role of a Membrane-Bound Dipeptidyl Aminopeptidase" pages 839-852**
**The file contains technical information submitted after the application was filed and not included in this specification**

(73) Proprietor: **Amgen Inc.**
**1900 Oak Terrace Lane**
**Thousand Oaks, California 91320 (US)**

(72) Inventor: **Bitter, Grant A.**
**3971 Calle Del Sol**
**Thousand Oaks California 01320 (US)**

(74) Representative: **Brown, John David et al**
**FORRESTER & BOEHMERT Widenmayerstrasse 4/I**
**D-8000 München 22 (DE)**

Courier Press, Leamington Spa, England.

## Description

The present invention relates to yeast cell transformation vectors.

Numerous substantial advances have recently been made in the use of recombinant DNA methodologies to secure the large scale microbial production of eukaryotic (e.g., mammalian) gene products in prokaryotic and eukaryotic cells grown in culture. In essence, these advances have generally involved the introduction into bacterial, yeast, and higher eukaryote "host" cell cultures of DNA sequences coding for polypeptides which wholly or partially duplicate the sequences of amino acids present in biologically active polypeptides ordinarily produced only in minute quantities by, e.g., specialized mammalian tissue cells. The hoped-for result of such introductions is the stable genetic transformation of the host cells so that the polypeptides coded for by the exogenous genes will be produced in quantity by the protein manufacturing apparatus of the cells.

It has long been the goal of workers in this field to devise methods and materials permitting not only the expression and stable accumulation of exogenous polypeptides of interest in host cells but also the secretory transport of intact polypeptide products from host cell cytoplasmic spaces into microbial periplasmic spaces or, preferably, outside the cell into the surrounding medium.

With particular regard to the use of *E. coli* bacterial cells as microbial hosts, it is known to attempt to secure expression of desired exogenous polypeptides as portions of so-called "fused" polypeptides including, e.g., endogenous enzymatic substances such as β-lactamase. Such enzymes normally migrate or are intracellularly processed toward *E. coli* periplasmic spaces and the fusion polypeptides including enzyme sequences are more or less readily isolated therefrom. See, Talmadge, et al., *PNAS (USA), 77,* 3369—3373 (1980). Extracellular chemical or enzymatic cleavage is employed to yield the desired exogenous polypeptides in purified form. See, e.g., U.S. Letters Patent No. 4,366,246 to Riggs. At present, no analogous methods have been found to be readily applicable to microbial synthetic procedures involving lower eukaryotic host cells such as yeast cells (e.g., *Saccharomyces cerevisiae*).

A considerable body of knowledge has developed concerning the manner in which mammalian gene products, especially small regulatory polypeptides, are produced. See, generally, Herbert, et al., *Cell, 30,* 1—2 (1982). As one example, biosynthetic studies have revealed that certain regulatory peptides are derived from precursor proteins which are ten times the size or more than the biologically active peptides. This fact indicates that significant intracellular processing must take place prior to secretion of discrete active products by the cells. The peptides must be cut out of the precursor and are sometimes chemically modified to active forms prior to secretion. Cleavage from precursors and chemical modifications such as glycosylation, phosphorylation and secretion are generally believed to occur in a well-defined order as newly synthesized proteins pass through the membranes of the endoplasmic reticulum, Golgi complexes, and vesicles prior to secretion of biologically active fragments.

Studies of polypeptides secreted by yeast cells have indicated that at least somewhat analogous processing of precursor proteins occurs prior to secretion into yeast cell periplasmic spaces or outside the yeast cell wall. A very recent review article on this subject by Schekman, et al., appears at pages 361—393 in "The Molecular Biology of the Yeast Saccharomyces, Metabolism and Gene Expression", Cold Spring Harbor Press (1982). Briefly put, the review article and the references cited therein indicate that eleven endogenous yeast polypeptide products have been identified which are secreted either into the periplasmic space or into the cellular medium or, on occasion, into both. Among the yeast polypeptides ordinarily secreted into the cellular growth medium are two yeast pheromones, mating factor α and *a*, pheromone peptidase, and "killer toxin". Among the yeast polypeptides ordinarily only transported to periplasmic spaces are invertase, L-asparaginase, and both the repressible and constitutive forms of acid phosphatase. Yeast products which have been isolated both from the periplasmic space and yeast cell culture medium include α-galactosidase, exo-1,3-β-glucanase, and endo-1,3-β-glucanase. The mechanisms which determine cell wall or extracellular location have not yet been elucidated.

The processing prior to secretion of certain of these polypeptides has been studied and it has generally been found that the products are initially expressed in cells in the form of precursor polypeptides having amino terminal regions including "signal" sequences (i.e., sequences of from 20—22 relatively hydrophobic amino acid residues belived to be functional in transport to the endoplasmic reticulum) and, in at least some instances, "pro" or "pre" sequences which are also ordinarily proteolytically cleaved from the portion of the precursor molecule to be secreted. See, Thill, et al., *Mol. & Cell. Biol, 3,* 570—579 (1983).

With the knowledge that yeast cells are capable of intracellular processing of endogenous precursor polypeptides in a manner analogous to the processing carried out in mammalian cell systems, stuudies were recently conducted concerning the potential for secretion of human interferons by yeast. See, Hitzeman, et al., *Science, 219,* 620—625 (1983). Briefly put, transformation vectors were constructed which included DNA sequences coding for synthesis of human interferons in the yeast *Saccharomyces cerevisiae*. It was reported that expression of interferon genes containing coding sequences for human "secretion signals" resulted in the secretion into the yeast cell culture medium of polypeptide fragments having interferon immunological activity. While the levels of interferon activity found in the medium were quite low and a significant percentage of the secreted material was incorrectly processed, the results of the studies were said to establish that lower eukaryotes such as yeast can rudimentarily utilize and intracellularly process human signal sequences in the manner of endogenous signal sequences.

Of particular interest to the background of the present invention is the developing body of information available concerning the synthesis and secretion of the yeast oligopeptide pheromone, or mating factor, commonly referred to as mating factor α ("MFα"). Mating in yeast appears to be facilitated by oligopeptide pheromones (mating factors) of two types, α and a, that cause the arrest of cells of the opposite type in the G1 phase of the cell division cycle. Yeast cells of the α mating type produce MFα in tridecapeptide and dodecapeptide forms which differ on the basis of the presence or absence of a terminal tryptophan residue, while cells of the a type produce MFa in two alternative undecapeptide forms which differ in terms of the identity of the sixth amino acid residue.

The structure of the yeast MFα gene has recently been the subject of study by Kurjan, et al., as reported in *Cell, 30*, 933—943 (1982). Briefly put, segments of yeast genomic DNA were inserted into a high copy number plasmid vector (YEp13). The vectors were employed to transform mutant matα2, leu2 yeast cells which failed to secrete MFα and the culture medium was assayed for the "restoration" of MFα secretory activity. Those plasmids including a 1.7kb EcoRI fragment together with one or more genomic EcoRI fragments of lesser size were able to restore MFα secretory function. Sequencing of portions of the 1.7 kb EcoRI fragment revealed that the cloned segment includes DNA sequences coding for four, spaced-apart copies of MFα within a putative precursor polypeptide which extends for a total of 165 amino acids.

The amino terminal region of the putative precursor delineated by Kurjan, et al., begins with a hydrophobic sequence of about 22 amino acids that presumably acts as a signal sequence for secretion. A following segment of approximately sixty amino acids contains three potential glycosylation sites. The carboxyl terminal region of the precursor contains four tandem copies of mature alpha factor, each preceded by "A spacer" peptides of six or eight amino acids, which are hypothesized to contain proteolytic processing signals.

The putative protein-coding region within the approximately 830 base pair sequence of the MFα gene published is as follows:

## TABLE I

```
1                    10                  20                  30                  40
•                    •                   o                   •                   •
ATG AGA TTT CCT TCA ATT TTT ACT GCA GTT TTA TTC GCA GCA
Met Arg Phe Pro Ser Ile Phe Thr Ala Val Leu Phe Ala Ala
1                                            10
               50                  60                  70                  80
               •                   •                   •                   •
TCC TCC GCA TTA GCT GCT CCA GTC AAC ACT ACA ACA GAA GAT
Ser Ser Ala Leu Ala Ala Pro Val Asn Thr Thr Thr Glu Asp
                        20
                    90                  100                 110                 120
                    •                   •                   •                   •
GAA ACG GCA CAA ATT CCG GCT GAA GCT GTC ATC GGT TAC TCA
Glu Thr Ala Gln Ile Pro Ala Glu Ala Val Ile Gly Tyr Ser
    30                                                      40
                130                 140                 150                 160
                •                   •                   •                   •
GAT TTA GAA GGG GAT TTC GAT GTT GCT GTT TTG CCA TTT TCC
Asp Leu Glu Gly Asp Phe Asp Val Ala Val Leu Pro Phe Ser
                                        50
170                 180                 190                 200                 210
o                   •                   o                   •                   •
AAC AGC ACA AAT AAC GGG TTA TTG TTT ATA AAT ACT ACT ATT
Asn Ser Thr Asn Asn Gly Leu Leu Phe Ile Asn Thr Thr Ile
                60                                                          70
                220                 230                 240                 250
                •                   •                   •                   •
GCC AGC ATT GCT GCT AAA GAA GAA GGG GTA TCT TTG GAT AAA
Ala Ser Ile Ala Ala Lys Glu Glu Gly Val Ser Leu Asp Lys
                                        80
            260      HindIII 270                      280                 290
            •          ____   •                       •                   •
AGA GAG GCT GAA GCT TGG CAT TGG TTG CAA CTA AAA CCT GGC
Arg Glu Ala Glu Ala Trp His Trp Leu Gln Leu Lys Pro Gly
                        90
        300                 310                 320           HindIII
        •                   •                   •            _____
CAA CCA ATG TAC AAG AGA GAA GCC GAA GCT GAA GCT TGG CAT
Gln Pro Met Tyr Lys Arg Glu Ala Glu Ala Glu Ala Trp His
                102                                                     111
    340                 350                 360                 370
    •                   •                   •                   •
TGG CTG CAA CTA AAG CCT GGC CAA CCA ATG TAC AAA AGA GAA
Trp Leu Gln Leu Lys Pro Gly Gln Pro Met Tyr Lys Arg Glu
                                            123
380                     HindIII             400                 410                 420
•                      _____             •                   •                   •
GCC GAC GCT GAA GCT TGG CAT TGG CTG CAA CTA AAG CCT GGC
Ala Asp Ala Glu Ala Trp His Trp Leu Gln Leu Lys Pro Gly
                        132   ·
            430                 440                 450      HindIII  460
            •                   •                   •        _____  •
CAA CCA ATG TAC AAA AGA GAA GCC GAC GCT GAA GCT TGG CAT
Gln Pro Met Tyr Lys Arg Glu Ala Asp Ala Glu Ala Trp His
                144                                                  153
        470                 480                 490
        •                   •                   •
TGG TTG CAG TTA AAA CCC GGC CAA CAA ATG TAC TAA
Trp Leu Gln Leu Lys Pro Gly Gln Pro Met Tyr Stop
                                        165
```

As previously noted, the MFα gene described in Kurjan, et al., *supra*, is contained on a 1.7 kilobase EcoRI yeast genomic fragment. Production of the gene product is inactivated by cleavage with the endonuclease HindIII and it was noted that HindIII digestion yielded small fragments generally including the following coding regions: α factor 1 (amino acids 90—102), spacer 2; α factor 2 (amino acids 111—123), spacer 3; α factor 3 (amino acids 132—144), spacer 4; spacer 1 and α factor 4 amino acids 153—165) remain on large fragments.

Thus, each MFα coding region in the carboxyl terminal coding region is preceded by a six or eight codon "spacer" coding region. The first of the spacers coded for has the sequence, —NH-Lys-Arg-Glu-Ala-Glu-Ala-COO—, while the second has the sequence, —NH-Lys-Arg-Glu-Ala-Glu-Ala-Glu-Ala-COO. The third and fourth spacers coded for have the same sequence of amino acid residues, i.e., —NH-Lys-Arg-Glu-Ala-Asp-Ala-Glu-Ala-COO—.

Among the proposals of Kurjan, et al. as to the mode of processing of the MFα precursor polypeptide leading up to secretion of MFα was that the precursor was targetted for processing in the endoplasmic reticulum by the putative 22 hydrophobic amino acid "signal" sequence in the amino terminal region of the precursor (amino acids 1—22). The post-targetting fate of the sequence was proteolytic cleavage from the remaining portions of the precursor. The following "pro" sequence of about 60 amino acids (residues 23—83) was proposed to be involved in subsequent targetting of the precursor for further processing and to an eventual fate similar to that of the "signal". Finally, it was proposed that the multiple copies of MFα were first separated by a trypsin-like enzymatic cleavage between the lysine and arginine residues at the beginning of each "spacer"; that the residual lysine at the carboxyl terminal of all but the fourth MFα copy was digested off by a yeast carboxy peptidase; and that diaminopeptidase enzymes would proteolytically delete the remaining "spacer" residues from the amino terminal of at least one of the four MFα copies.

While the work of Kurjan, et al. served to provide much valuable information and many valuable proposals concerning MFα synthesis and secretion in yeast, many questions significant to application of the information to systems other than those specifically involving MFα secretion remained unanswered. Among these was whether the above-noted 1.7kb EcoRI yeast genome fragment provides a self-contained sequence capable of directing synthesis of MFα (i.e., whether it included the entire endogenous promoter/regulator for precursor synthesis or, on the other hand, required the presence of other DNA sequences). Other unanswered questions included whether the presence of DNA "repeats" was required for MFα expression, whether the specific size of the MFα polypeptide is a critical factor in secretory processing events, and whether all potential copies of MFα in the precursor polypeptide are in fact secreted by yeast cells.

A recent publication by Julius, et al., *Cell, 32*, 839—852 (1983) serves to partially confirm the MFα precursor hypothesis of Kurjan, et al. in noting that mutant yeast strains defective in their capacity to produce certain membrane-bound, heat-stable dipeptidyl diaminopeptidase enzymes (coded for by the "ste13" gene) secrete incompletely processed forms of MFα having additional amino terminal residues duplicating "spacer" sequences described by Kurjan, et al. Restoration of the mutants' capacity to properly process MFα was demonstrated upon transformation of cells with plasmid-borne copies of the non-mutant form of the ste13 gene.

From the above description of the state of the art, it will be apparent that there continues to exist a need in the art for methods and materials for securing microbial expression of exogenous polypeptide products accompanied by some degree of intracellular secretory processing of products facilitating the isolation of products in purified form. Despite varying degrees of knowledge concerning synthesis and processing of yeast-secreted polypeptides and despite some preliminary success in procedures involving yeast secretory processing of exogenous gene products in the form of exogenous precursor polypeptides, the art has been provided with no procedures which take joint advantage of yeast cell capacities both to synthesize exogenous gene products and to properly process endogenous precursor polypeptides in a manner permitting exogenous gene products to be secreted by transformed yeast cells.

DNA transformation vectors of the invention are plasmids pYαE and pYcαE on deposit under contract with the American Type Culture Collection, Rockville, Maryland, as ATCC Nos. 40068 and 40069, respectively. Both these plasmids include hybrid polypeptide coding regions under control of promoter/regulator sequences duplicating those associated with genomic expression of MFα by yeast cells. Plasmid pYαE (ATCC No. 40068) may be employed according to the present invention to transform a suitable *Saccharomyces cerevisiae* cell line (e.g., any α, leu2 strain such as GM3C—2) and the cultured growth of cells so transformed results in the accumulation, in the medium of cell growth, of polypeptide products possessing one or more of the biological activities (e.g., immunoreactivity) of human β-endorphin.

Other aspects and advantages of the invention will become apparent upon consideration of the following detailed description of preferred embodiments thereof.

Detailed Description

The following examples relate to manipulations involved in securing yeast cell synthesis and secretion of polypeptide substances having one or more of the biological activities of human β-endorphin. More specifically, Examples 1 through 7 relate to: (1) the isolation of an MFα structural gene as a DNA fragment from a yeast genomic library and the partial sequencing of the cloned fragment; (2) the construction of a DNA sequence coding for human β-endorphin; (3) the ligation of the β-endorphin coding DNA sequence

into the MFα structural gene; (4) the insertion of the resulting DNA sequence into a transformation vector; (5) the transformation of yeast cells with the resulting vector; (6) the isolation and characterization of polypeptide products secreted into the culture medium by transformed cells; and (7) the construction of an alternative transformation vector.

## Example 1

A *Saccharomyces cerevisiae* genome library in *E. coli* was screened with a synthetic oligonucleotide hybridization probe, and a plasmid with complementarity to the probe was cloned. From this cloned plasmid a 2.1kb EcoRI fragment with complementarity to the probe was subcloned in pBR322. The oligonucleotide probe used duplicates the sequence of bases later designated 474 through 498 of the sense strand DNA sequence set out in Figure 5 of Kurjan, et al., *supra*. Approximately 500 base pairs of the isolated fragment were initially sequenced by Maxam-Gilbert and dideoxy chain termination techniques and found to be essentially identical to the sequence of the protein coding region of an MFα structural gene set out by Kurjan, et al., *supra*. The 2.1kb fragment was digested with XbaI. The larger, 1.7kb digestion fragment obtained was ligated to a BamHI "linker" DNA sequence and inserted into an *E. coli* bacterial plasmid (pBRΔH, i.e., pBR322 which had been modified to delete the HindIII site) cut with BamHI. The resulting plasmid, designated pαFc, was amplified.

## Example 2

A DNA sequence coding for human [Leu⁵] β-endorphin polypeptide was synthesized and constructed according to the procedures of co-pending U.S. Patent Application Serial No. 375,493 filed May 6, 1982 by Stabinsky. The specific sequence constructed is set out in Table II below. Terminal base pair sequences outside the coding region are provided to facilitate insertion into the MFα structural gene as described, *infra*.

## TABLE II

HindIII
```
        Tyr Gly Gly Phe Leu Thr Ser Glu Lys Ser Gln Thr
   AGCT TAC GGT GGT TTC TTG ACC TCT GAA AAG TCT CAA ACT
        ATG CCA CCA AAG AAC TGG AGA CTT TTC AGA GTT TGA


   Pro Leu Val Thr Leu Phe Lys Asn Ala Ile Ile Lys Asn Ala
   CCA TTG GTT ACT TTG TTC AAG AAC GCT ATC ATC AAG AAC GCT
   GGT AAC CAA TGA AAC AAG TTC TTG CGA TAG TAG TTC TTG CGA


   Tyr Lys Lys Gly Glu Ter Ter
   TAC AAG AAG GGT GAA TAA TAA GCTTG
   ATG TTC TTC CCA CTT ATT ATT CGAACCTAG
```
                          HindII   BamHI

The constructed sequence was cloned into the Rf M13mp9 which had been cut with HindIII and BamHI and the sequence was confirmed. The resulting Rf M13 DNA, designated M13/βEnd-9, was purified.

## Example 3

Plasmid pαFc was digested with HindIII to delete three of the four MFα coding regions. As may be noted from the sequence of the protein-coding region of the MFα structural gene in Table I, after such endonculease treatment there remained a HindIII sticky end at the terminal portion of the first of the "spacer" amino acid sequences (Ala⁸⁹) and a HindIII sticky end just before the final MFα sequence (Trp¹⁵³).

M13/βEnd-9, containing the [Leu⁵] β-endorphin gene, was similarly digested with HindIII and the resulting 107 base pair fragment was purified and ligated into the HindIII cleaved pαFc to generate plasmid pαE. The DNA sequence thus generated is seen to code for synthesis of a new hybrid polypeptide. In the new hybrid polypeptide, there is included, in the carboxyl terminal portion, an exogenous polypeptide, i.e. [Leu⁵] β-endorphin. In the new hybrid polypeptide, there are included sequences of amino acid residues duplicative of one or more sequences which are extant in the amino terminal region of an endogenous polypeptide precursor of a selected yeast-secreted polypeptide (i.e., MFα) and which are normally proteolytically cleaved from the yeast-secreted polypeptide portion of the precursor prior to secretion.

It may be here noted that in an alternative construction available according to the invention, a tandem repeating β-endorphin gene or other selected gene might be constructed and inserted into the HindIII cleaved PαFc. In such a tandem repeating gene construction, the termination codons of the first β-endorphin coding sequence would be deleted and the first coding sequence would be separated from the second sequence by, e.g., a DNA sequence coding for part or all of one of the alternative MFα "spacer"

6

polypeptide forms. It would be preferred that alternative codons be employed in the region joining the spacer to the second β-endorphin sequence so that no HindIII restriction site would remain. Upon insertion as above, the novel DNA sequence would code for a hybrid polypeptide which further included a normally proteolytically cleaved endogenous yeast sequence in its carboxyl terminal region, i.e., between two β-endorphin analog polypeptides. Similarly, multiple repeats of a selected exogenous gene may be incorporated separately by part or all of any of the variant spacers.

Example 4

Plasmid pαE was digested with BamHI and the small fragment obtained was ligated into a high copy number yeast/*E. coli* shuttle vector pGT41 (cut with BamHI) to form plasmid pYαE (ATCC No. 40068) which was amplified in *E. coli.*

Example 5

Plasmid pYαE was employed to transform a suitable α, Leu2⁻ strain of *Saccharomyces cerevisiae* (GM3C—2) wherein the Leu2⁺ phenotype allowed selection of transformants. Transformed cells were grown in culture at 30°C in 0.67 Yeast Nitrogen Base without amino acids (Difco), 2% glucose, 1% histidine and 1% tryptophan. Additionally, strain GM3C—2 transformed with a plasmid identical to pYαE, with the exception that the β-endorphin gene was in the opposite orientation, was cultured under identical conditions as a control.

Example 6

Cultures from transformed and control cells were collected, centrifuged, and the supernatants tested for the presence of β-endorphin activity bgy means of a competitive radioimmunoassay for human β-endorphin [New England Nuclear Catalog No. NEK—003]. No activity at all was determined in the control media, while significant β-endorphin activity, on an order representing 200 micrograms of product per O.D. liter, was found in the media from cultured growth of transformed cells.

HPLC analysis of the concentrated active media revealed three major RIA activity peaks. The most prominent peak, representing approximately one-third of the total β-endorphin activity, was isolated and amino acid sequencing revealed an essentially pure preparation of a polypeptide duplicating the sequence of the final 12 amino acid residues of human β-endorphin. Experimental procedures are under way to determine whether the 12 amino acid product is the result of intracellular proteolytic processing by the transformed cells or is an artifact generated by extracellular proteolytic cleavage occurring during handling of the culture medium. If the latter proves to be the case, protease inhibitors will be added to the medium in future isolative processing.

Example 7

In order to determine whether secretory processing of yeast synthesized β-endorphin analog by transformed cells will be facilitated by reduction of the quantities of hybrid polypeptide produced, a single copy ("centromere") plasmid pYcαE (ATCC No. 40069) has been constructed with an inserted BamHI fragment from pαE. Analysis of cell media of yeast transformed with this vector is presently under way.

In further experimental studies, the potential secretory rate limiting effects of available secretory processing enzymes will be determined. In one such procedure, yeast cells transformed with vectors of the invention will also be transformed to incorporate an ste13 gene as described in Julius, et a., *supra*, so as to provide over-production of the heat stable dipeptidyl aminopeptidase believed to be involved in MFα secretory processing.

**Claims**

1. A yeast cell transformation vector which is plasmid pYαE, ATCC No. 40068.
2. A yeast cell transformation vector which is plasmid pYcαE, ATCC No. 40069.

**Patentansprüche**

1. Vektor zur Transformation von Hefe, welcher Plasmid pYαE, ATCC No. 40068 ist.
2. Vektor zur Transformation von Hefe, welcher Plasmid pYcαE, ATCC No. 40069 ist.

**Revendications**

1. Vecteur de transformation de la levure qui est le plasmide pYαE, ATCC N° 40068.
2. Vecteur de transformation de la levure qui est le plasmide pYcαE, ATCC N° 40069.